# EUROPEAN PATENT APPLICATION

(11) **EP 4 837 509 A1**
(43) Date of publication of application: **11.11.2026**
(21) Application number: 25768588.3
(22) Date of filing: 07.03.2025
(51) Int. Cl.: A61B 6/00, A61B 6/50, A61B 6/03, G06T 7/11

(54) **APPARATUS AND METHOD FOR MEASURING MUSCLE FOR EACH PART AND DETECTING VERTEBRAL SEGMENT IN MEDICAL IMAGE**

(30) Priority: 08.03.2024 KR 20240033065; 08.03.2024 KR 20240033068
(71) Applicant: Promedius Inc., Seoul 05510 (KR)
(72) Inventor: PARK, Junhyuk, Seoul 06932 (KR); JEONG, Jinhoon, Seoul 05310 (KR)
(74) Representative: Studio Torta S.p.A.
(86) International application number: PCT/KR2025/099625
(87) International publication number: WO 2025/188169

(57) **Abstract**

The present disclosure relates to an apparatus and method for measuring muscles by region and detecting vertebral levels in medical images. The apparatus may include a memory configured to store at least one process for performing measurement of muscles by region for an L3 slice in a CT image and a processor configured to perform an operation according to the at least one process. The processor may generate a first muscle mask and a second muscle mask, respectively, indicate an intersection region of the first muscle mask and the second muscle mask as an actual muscle, and divide the actual muscle into a plurality of regions to represent a muscle quality for each region.

## Description

### [TECHNICAL FIELD]

The present disclosure relates to an apparatus and method for measuring muscles by region and detecting vertebral levels in medical images.

### [BACKGROUND ART]

Recently, the development of artificial intelligence technology has been widely utilized to indicate specific regions, such as diseases or muscles, in medical images.

Sarcopenia refers to a disease in which muscle mass and muscle function decrease with aging. The force for supporting bones and joints becomes weakened, thereby increasing pressure applied to the skeletal system, aggravating joint pain, reducing stability, and increasing the risk of falls and fractures. In addition, the incidence of diabetes, hypertension, and cardiovascular diseases also increases, and secondary diseases may occur, such as dysphagia of digestive organs composed of muscles.

Such sarcopenia is comprehensively diagnosed through a body composition analysis, a muscle mass measurement method for checking muscles of the thigh, a muscle strength evaluation method for evaluating grip strength or lower extremity muscle strength, and a physical activity capability evaluation method such as walking or standing up from a seated position.

Due to the development of artificial intelligence, AI-based muscle mass measurement algorithms based on images such as MRI, CT, and ultrasound images have recently been developed, and there is a need for a method capable of assisting early diagnosis and follow-up of sarcopenia by utilizing such technologies.

In addition, the recent development of artificial intelligence technology has also been used for finding specific structures in medical images. When performing activities such as measuring muscle mass, physicians typically identify a slice based on a vertebral level and then interpret the corresponding slice.

In CT, there is a Maximum Intensity Projection (MIP) technique as preprocessing for clearly viewing bones. This is a three-dimensional technique that displays only the highest HU value among voxels constituting an image. When a projection angle is designated in a direction in which reconstructed images (Transvers axial image) for obtaining a three-dimensional image are to be observed, only a voxel having the highest HU value among voxels arranged in parallel in the designated direction is selected and displayed.

A technique for finding a specific vertebral level through MIP has already been disclosed in papers and the like. However, this technique has a problem in that performance is degraded when there are many artifacts (calcified blood vessels, metal pins, etc.) that appear bright in CT or when image quality of the vertebral portion is low for various reasons.

### [DETAILED DESCRIPTION OF THE INVENTION]

### [TECHINICAL PROBLEM]

An object of embodiments disclosed in the present disclosure is to provide an apparatus and method capable of measuring muscles by region and detecting vertebral levels in medical images.

Specifically, an object of embodiments disclosed in the present disclosure is to provide an apparatus and method capable of measuring muscles by region for an L3 slice in a CT image.

Further, specifically, an object of embodiments disclosed in the present disclosure is to provide an apparatus and method capable of detecting vertebral levels in an AI-based MIP image.

### [TECHNICAL SOLUTION]

According to an embodiment of the present disclosure for achieving the above-described technical problem, an apparatus for measuring muscles by region for an L3 slice in a CT image may include a memory configured to store at least one process for performing measurement of muscles by region for the L3 slice in the CT image and a processor configured to perform an operation according to the at least one process, wherein the processor may generate a first muscle mask and a second muscle mask, respectively, indicate an intersection region of the first muscle mask and the second muscle mask as an actual muscle, and divide the actual muscle into a plurality of regions to represent a muscle quality for each region.

Further, the first muscle mask may be extracted from an L3 slice image based on a whole-muscle segmentation model, and the second muscle mask may be extracted from the L3 slice image based on a regional muscle segmentation model.

Further, the plurality of regions may include an anterior muscle, a lateral muscle, a posterior muscle, and a psoas muscle.

Further, the whole-muscle segmentation model and the regional muscle segmentation model may have different ranges of regions of interest.

Further, according to an embodiment of the present disclosure for achieving the above-described technical problem, a method for measuring muscles by region for an L3 slice in a CT image, performed by a processor of an apparatus, may include generating a first muscle mask and a second muscle mask, respectively; indicating an intersection region of the first muscle mask and the second muscle mask as an actual muscle; and dividing the actual muscle into a plurality of regions to represent a muscle quality for each region.

Further, according to an embodiment of the present disclosure for achieving the above-described technical problem, an apparatus for detecting vertebral levels in an AI-based MIP image may include a memory configured to store at least one process for performing AI-based vertebral level detection in the MIP image and a processor configured to perform an operation according to the at least one process, wherein the processor may divide the MIP image into a first region and a second region using an AI-based segmentation model, remove portions other than a vertebral portion from each of the MIP image of the first region and the MIP image of the second region, infer an inference value for each vertebral level from the MIP image of the first region and the MIP image of the second region in which only the vertebral portion remains using an AI-based detection model, and determine a final slice through the inference value.

Further, the first region may be a sagittal plane region, and the second region may be a coronal plane region.

Further, the detection model may include a first detection model for the first region and a second detection model for the second region.

Further, the processor may find a specific vertebral level from the MIP image of the first region and the MIP image of the second region in which only the vertebral portion remains, and may determine the final slice using an inference value of the first detection model and an inference value of the second detection model for the specific vertebral level.

Further, according to an embodiment of the present disclosure for achieving the above-described technical problem, a method for detecting vertebral levels in an AI-based MIP image, performed by a processor of an apparatus, may include dividing the MIP image into a first region and a second region using an AI-based segmentation model; removing portions other than a vertebral portion from each of the MIP image of the first region and the MIP image of the second region; inferring an inference value for each vertebral level from the MIP image of the first region and the MIP image of the second region in which only the vertebral portion remains using an AI-based detection model; and determining a final slice through the inference value.

### [ADVANTAGEOUS EFFECTS OF THE INVENTION]

According to the above-described means for solving the technical problem of the present disclosure, performance of a conventional method of segmenting muscles by region in an L3 slice of a CT image to represent qualitative characteristics for each region can be improved.

Further, by improving performance of an algorithm capable of representing muscle quality and muscle quantity for respective detailed regions in a CT image, development of a personalized treatment plan can be assisted through quantitative indicators for respective regions of an individual.

Further, in particular, in order to develop a personalized treatment plan for an individual patient, it is possible to identify which muscle region of the individual patient has a problem, and thus this can play an important role in developing a personalized treatment plan for the individual patient.

Further, according to the above-described means for solving the technical problem of the present disclosure, performance of a conventional technique using MIP to find a specific vertebral level in CT can be improved. Specifically, performance of an artificial intelligence model for finding a slice corresponding to a location of a specific vertebral level in an abdomen CT can be improved.

Further, according to the above-described means for solving the technical problem of the present disclosure, in detecting vertebral levels, an algorithm having excellent performance capable of finding a slice corresponding to a vertebral level more accurately can be provided even in a low-quality CT image in which a vertebral level cannot be detected or may be incorrectly measured by conventional techniques.

The effects of the present disclosure are not limited to the above-described effects, and other effects not described above will be clearly understood by those skilled in the art from the following description.

### [DESCRIPTION OF THE DRAWINGS]

FIG. 1 is a block diagram illustrating an apparatus for measuring muscles by region and detecting vertebral levels in medical images according to an embodiment of the present disclosure.
FIG. 2 is a flowchart illustrating a process of measuring muscles by region for an L3 slice in a CT image according to an embodiment of the present disclosure.
FIG. 3 is a diagram for describing a process of generating images for respective muscles according to an embodiment of the present disclosure.
FIG. 4 is a flowchart illustrating a process of detecting vertebral levels in an AI-based MIP image according to an embodiment of the present disclosure.
FIG. 5 is a diagram for describing a process of determining a final slice through an inference value for each vertebral level according to an embodiment of the present disclosure.

### [BEST MODE]

Throughout the present disclosure, like reference numerals refer to like elements. The present disclosure does not describe all elements of the embodiments, and descriptions generally known in the technical field to which the present disclosure pertains or redundant descriptions among the embodiments are omitted. As used herein, the terms "unit," "module," "member," and "block" may be implemented in software or hardware. According to embodiments, a plurality of "units," "modules," "members," or "blocks" may be implemented as a single element, or a single "unit," "module," "member," or "block" may include a plurality of elements. Throughout the specification, when a portion is described as being "connected" to another portion, this includes not only a case in which the portion is directly connected thereto, but also a case in which the portion is indirectly connected thereto, and such indirect connection includes connection through a wireless communication network.

Further, when a portion is described as "including" a certain element, this means that the portion may further include another element rather than excluding another element unless otherwise specifically stated.

Throughout the specification, when a member is described as being positioned "on" another member, this includes not only a case in which the member is in contact with the other member, but also a case in which another member is interposed therebetween.

The terms "first," "second," and the like are used to distinguish one element from another element, and the elements are not limited by the foregoing terms.

A singular expression includes a plural expression unless the context clearly indicates otherwise.

In each step, reference signs are used for convenience of description, and the reference signs do not describe the order of the respective steps. Accordingly, unless a specific order is clearly stated in the context, the respective steps may be performed in an order different from the stated order.

Hereinafter, the operating principle and embodiments of the present disclosure will be described with reference to the accompanying drawings.

Prior to the description, the meanings of terms used in the present specification will be briefly described. However, the descriptions of the terms are provided to facilitate understanding of the present specification, and it should be noted that the descriptions are not intended to limit the technical spirit of the present disclosure unless explicitly stated as limiting the present disclosure.

In the present specification, the term "apparatus" includes all types of devices capable of performing arithmetic processing and providing results to a user. For example, the apparatus may include a computer, a server apparatus, and a portable terminal, or may be any one thereof.

Here, the computer may include, for example, a notebook equipped with a WEB Browser, a desktop, a laptop, a tablet PC, and a slate PC.

The server apparatus is a server that processes information by communicating with an external device and may include an application server, a computing server, a database server, a file server, a game server, a mail server, a proxy server, and a web server.

The portable terminal may include, for example, a wireless communication device having portability and mobility, including all types of handheld-based wireless communication devices such as a PCS (Personal Communication System), GSM (Global System for Mobile Communications), PDC (Personal Digital Cellular), PHS (Personal Handyphone System), PDA (Personal Digital Assistant), IMT (International Mobile Telecommunication)-2000, CDMA (Code Division Multiple Access)-2000, W-CDMA (Wideband Code Division Multiple Access), a WiBro (Wireless Broadband Internet) terminal, and a smart phone, as well as wearable devices such as a watch, a ring, a bracelet, an anklet, a necklace, glasses, contact lenses, or a head-mounted device (HMD).

Functions related to artificial intelligence according to the present disclosure operate through a processor and a memory. The processor may include one or more processors. The one or more processors may be a general-purpose processor such as a CPU, an AP, or a DSP (Digital Signal Processor), a graphics-dedicated processor such as a GPU or a VPU (Vision Processing Unit), or an artificial intelligence-dedicated processor such as an NPU. The one or more processors control processing of input data according to predefined operation rules or an artificial intelligence model stored in the memory. Alternatively, when the one or more processors are artificial intelligence-dedicated processors, the artificial intelligence-dedicated processors may be designed with a hardware architecture specialized for processing a specific artificial intelligence model.

The predefined operation rules or the artificial intelligence model are characterized by being generated through learning. Here, being generated through learning means that a predefined operation rule or an artificial intelligence model configured to perform a desired characteristic (or objective) is generated by training a basic artificial intelligence model using a plurality of training data through a learning algorithm. Such learning may be performed by a device in which the artificial intelligence according to the present disclosure is executed or may be performed through a separate server and/or system. Examples of the learning algorithm include supervised learning, unsupervised learning, semi-supervised learning, and reinforcement learning, but are not limited to the foregoing examples.

The artificial intelligence model may include a plurality of neural network layers. Each of the plurality of neural network layers has a plurality of weight values and performs a neural network operation through an operation between an operation result of a previous layer and the plurality of weight values. The plurality of weight values included in the plurality of neural network layers may be optimized according to a learning result of the artificial intelligence model. For example, during a learning process, the plurality of weight values may be updated so that a loss value or a cost value acquired by the artificial intelligence model is reduced or minimized. The artificial neural network may include a Deep Neural Network (DNN), such as a CNN (Convolutional Neural Network), a DNN (Deep Neural Network), an RNN (Recurrent Neural Network), an RBM (Restricted Boltzmann Machine), a DBN (Deep Belief Network), a BRDNN (Bidirectional Recurrent Deep Neural Network), or a Deep Q-Network, but is not limited to the foregoing examples.

The processor may generate a neural network, train (learn) the neural network, perform an operation based on received input data, generate an information signal based on an operation result, or retrain the neural network.

The neural network may include a CNN (Convolutional Neural Network), an RNN (Recurrent Neural Network), a perceptron, a multilayer perceptron, an FF (Feed Forward), an RBF (Radial Basis Function Network), a DFF (Deep Feed Forward), an LSTM (Long Short-Term Memory), a GRU (Gated Recurrent Unit), an AE (Auto Encoder), a VAE (Variational Auto Encoder), a DAE (Denoising Auto Encoder), an SAE (Sparse Auto Encoder), an MC (Markov Chain), an HN (Hopfield Network), a BM (Boltzmann Machine), an RBM (Restricted Boltzmann Machine), a DBN (Deep Belief Network), a DCN (Deep Convolutional Network), a DN (Deconvolutional Network), a DCIGN (Deep Convolutional Inverse Graphics Network), a GAN (Generative Adversarial Network), an LSM (Liquid State Machine), an ELM (Extreme Learning Machine), an ESN (Echo State Network), a DRN (Deep Residual Network), a DNC (Differentiable Neural Computer), an NTM (Neural Turing Machine), a CN (Capsule Network), a KN (Kohonen Network), and an AN (Attention Network), but is not limited thereto, and those skilled in the art will understand that any neural network may be included.

According to an exemplary embodiment of the present disclosure, the processor may use various artificial intelligence architectures and algorithms, including a CNN (Convolution Neural Network) such as GoogleNet, AlexNet, and VGG Network, an R-CNN (Region with Convolution Neural Network), an RPN (Region Proposal Network), an RNN (Recurrent Neural Network), an S-DNN (Stacking-based Deep Neural Network), an S-SDNN (State-Space Dynamic Neural Network), a Deconvolution Network, a DBN (Deep Belief Network), an RBM (Restricted Boltzmann Machine), a Fully Convolutional Network, an LSTM (Long Short-Term Memory) Network, a Classification Network, Generative Modeling, eXplainable AI, Continual AI, Representation Learning, AI for Material Design, BERT, SP-BERT, MRC/QA, Text Analysis, Dialog System, an LLM (Large Language Model), Generative AI, GPT (Generative Pre-trained Transformer) for natural language processing, Visual Analytics, Visual Understanding, Video Synthesis, ResNet, Anomaly Detection, Prediction, Time-Series Forecasting, Optimization, Recommendation, and Data Creation for data intelligence, but is not limited thereto.

The present disclosure relates to a method for representing qualitative characteristics for respective muscle regions when segmenting muscles and evaluating muscle quality in an abdomen CT image.

Hereinafter, embodiments of the present disclosure will be described in detail with reference to the accompanying drawings.

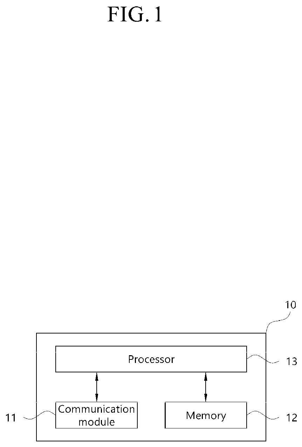

FIG. 1 is a block diagram illustrating an apparatus for measuring muscles by region and detecting vertebral levels in medical images according to an embodiment of the present disclosure.

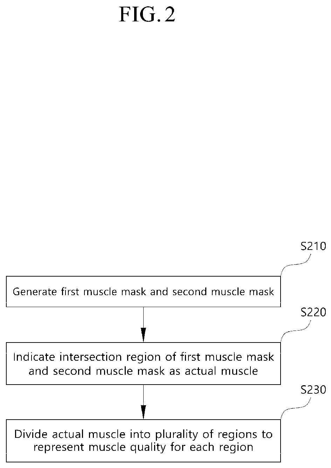

FIG. 2 is a flowchart illustrating a process of measuring muscles by region for an L3 slice in a CT image according to an embodiment of the present disclosure.

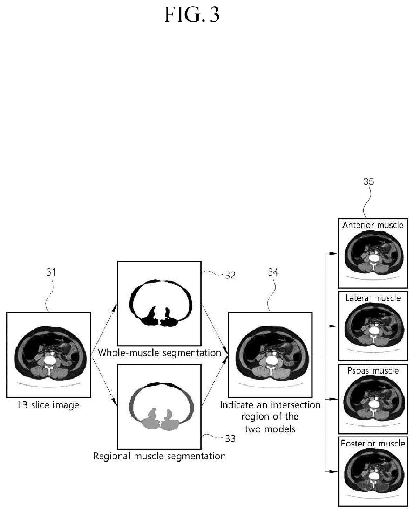

FIG. 3 is a diagram for describing a process of generating images for respective muscles according to an embodiment of the present disclosure.

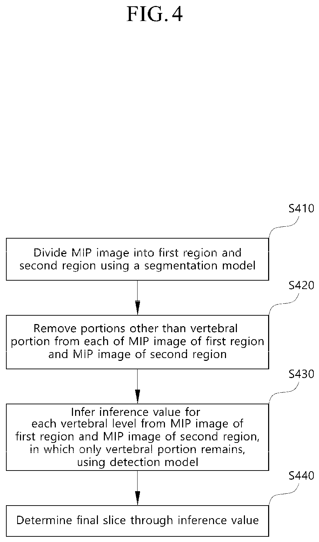

FIG. 4 is a flowchart illustrating a process of detecting vertebral levels in an AI-based MIP image according to an embodiment of the present disclosure.

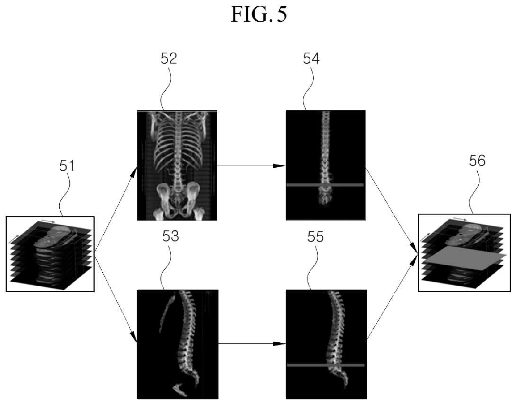

FIG. 5 is a diagram for describing a process of determining a final slice through an inference value for each vertebral level according to an embodiment of the present disclosure.

The apparatus according to the present disclosure may measure muscles by region for an L3 slice in a CT image and may detect vertebral levels in an AI-based MIP image.

An apparatus 10 according to an embodiment of the present disclosure may include a communication module 11, a memory 12, and a processor 13. However, in some embodiments, the apparatus 10 may include fewer components or more components than those illustrated in FIG. 1.

The communication module 11 may include one or more modules configured to enable wireless or wired communication between the apparatus 10 and an external device (not shown) and between the apparatus 10 and a communication network. For example, the communication module 11 may include at least one of a wired communication module, a wireless communication module, a short-range communication module, and a location information module.

Various types of communication networks may be used as the communication network. For example, a wireless communication scheme such as WLAN (Wireless LAN), Wi-Fi, WiBro, WiMAX, or HSDPA (High Speed Downlink Packet Access), or a wired communication scheme such as Ethernet, xDSL (ADSL, VDSL), HFC (Hybrid Fiber Coax), FTTC (Fiber To The Curb), or FTTH (Fiber To The Home) may be used.

Meanwhile, the communication network is not limited to the communication schemes described above and may include any other communication scheme generally known or developed in the future in addition to the communication schemes described above.

The wired communication module may include not only various wired communication modules such as a Local Area Network (LAN) module, a Wide Area Network (WAN) module, and a Value Added Network (VAN) module, but also various cable communication modules such as USB (Universal Serial Bus), HDMI (High Definition Multimedia Interface), DVI (Digital Visual Interface), RS-232 (Recommended Standard 232), power line communication, and POTS (Plain Old Telephone Service).

The wireless communication module may include, in addition to a Wi-Fi module and a WiBro (Wireless Broadband) module, a wireless communication module supporting various wireless communication schemes such as GSM (Global System for Mobile Communication), CDMA (Code Division Multiple Access), WCDMA (Wideband Code Division Multiple Access), UMTS (Universal Mobile Telecommunications System), TDMA (Time Division Multiple Access), LTE (Long Term Evolution), 4G, 5G, and 6G.

The short-range communication module is for short-range communication and may support short-range communication using at least one of Bluetooth^{™}, RFID (Radio Frequency Identification), Infrared Data Association (IrDA), UWB (Ultra Wideband), ZigBee, NFC (Near Field Communication), Wi-Fi (Wireless Fidelity), Wi-Fi Direct, and Wireless USB (Wireless Universal Serial Bus).

According to an embodiment of the present disclosure, the memory 12 may store at least one process for performing measurement of muscles by region for an L3 slice in a CT image, and the processor 13 may perform measurement of muscles by region for the L3 slice in the CT image according to the at least one process.

Further, according to an embodiment of the present disclosure, the memory 12 may store at least one process for performing AI-based detection of vertebral levels in an MIP image, and the processor 13 may perform AI-based detection of vertebral levels in the MIP image according to the at least one process.

The memory 12 may store data supporting various functions of the apparatus 10, programs for operations of the processor 13, input/output data (for example, music files, still images, and moving images), a plurality of application programs (applications) executed in the apparatus 10, data for operations of the apparatus 10, and instructions. At least some of the application programs may be downloaded from an external server through wireless communication.

The memory 12 may include at least one type of storage medium selected from a flash memory type, a hard disk type, an SSD (Solid State Disk) type, an SDD (Silicon Disk Drive) type, a multimedia card micro type, a card-type memory (for example, an SD memory or an XD memory), RAM (Random Access Memory), SRAM (Static Random Access Memory), ROM (Read-Only Memory), EEPROM (Electrically Erasable Programmable Read-Only Memory), PROM (Programmable Read-Only Memory), a magnetic memory, a magnetic disk, and an optical disk. Further, the memory 12 may be a database separated from the muscle measurement apparatus 10 and connected thereto through wired or wireless communication.

The processor 13 may perform the above-described operations using a memory storing data for an algorithm or a program implementing the algorithm for controlling operations of the components in the apparatus 10 and data stored in the memory. The memory 12 and the processor 13 may be implemented as separate chips, respectively. Alternatively, the memory 12 and the processor 13 may be implemented as a single chip.

Further, in order to implement various embodiments of the present disclosure described below with reference to FIGS. 2 to 5 in the apparatus 10, the processor 13 may control any one or a combination of a plurality of the components described above.

Hereinafter, a process of measuring muscles by region for an L3 slice in a CT image according to an embodiment of the present disclosure will be described in detail with reference to FIGS. 2 and 3.

Referring to FIG. 2, the processor 13 may generate a first muscle mask and a second muscle mask, respectively (S210).

The processor 13 may indicate an intersection region of the first muscle mask and the second muscle mask as an actual muscle (S220).

The processor 13 may divide the actual muscle into a plurality of regions and represent a muscle quality for each region (S230).

Here, the first muscle mask may be an image extracted from an L3 slice image based on a first model for segmenting an entire muscle based on artificial intelligence, and the second muscle mask may be an image extracted from the L3 slice image based on a second model for segmenting muscles of detailed regions based on artificial intelligence.

Here, the plurality of regions may include an anterior muscle, a lateral muscle, a posterior muscle, and a psoas muscle.

That is, the processor 13 may generate the first muscle mask by inputting the L3 slice image to the first model to extract the first muscle mask, and may generate the second muscle mask by inputting the L3 slice image to the second model to extract the second muscle mask.

The processor 13 may indicate that an intersection region of the first muscle mask and the second muscle mask is an actual muscle and may divide the indicated actual muscle into a plurality of regions (regions included in detailed region segmentation) to represent a muscle quality for each region.

Here, the first model and the second model are independently trained and may have different ranges of regions of interest.

Referring to FIG. 3, the processor 13 may extract a first muscle mask 32 as a detailed result for a muscle region from an entire image of an L3 slice image 31 through the first model.

The processor 13 may extract a second muscle mask 33 through the first model, the second muscle mask 33 distinguishing regions corresponding to an anterior muscle, a lateral muscle, a posterior muscle, and a psoas muscle from the muscle region of the L3 slice image 31.

At this time, the processor 13 may ensemble (combine) the first model and the second model to extract an image 33 representing an intersection region of the first model and the second model. That is, measurement accuracy of actual muscles by region can be improved for the intersection region.

The ensemble technique used herein may include methodologies such as voting, bagging, and boosting.

The processor 13 may extract an image 34 visualizing a muscle quality and a muscle quantity for each region based on an ensemble result of the first model and the second model.

That is, the processor 13 may project the images 32 and 33 corresponding to results respectively inferred through the first model and the second model onto the original image 31 to verify whether corresponding portions are actual muscles and may visualize and provide the muscle quality and the muscle quantity for each region based on regions subdivided through the inference process using the second model.

Although FIG. 2 illustrates that the respective steps are sequentially performed, this is merely for illustratively describing the technical spirit of the present embodiment. Accordingly, those skilled in the art may variously modify and apply the embodiment by changing the order described in FIG. 2 or by performing the respective steps in parallel without departing from the essential characteristics of the present embodiment. Therefore, FIG. 2 is not limited to a chronological order.

Meanwhile, in the above description, the steps illustrated in FIG. 2 may be further divided into additional steps or combined into fewer steps according to an implementation of the present disclosure. Further, some steps may be omitted as necessary, and the order of the respective steps may be changed.

Meanwhile, the disclosed embodiments may be implemented in the form of a recording medium storing instructions executable by a computer. The instructions may be stored in the form of program code, and when executed by a processor, may generate program modules to perform operations of the disclosed embodiments. The recording medium may be implemented as a computer-readable recording medium.

Hereinafter, a process of detecting vertebral levels in an AI-based MIP image according to an embodiment of the present disclosure will be described in further detail with reference to FIGS. 4 and 5.

Referring to FIG. 4, the processor 13 may divide an MIP image into a first region and a second region using an AI-based segmentation model (S410).

The processor 13 may remove portions other than a vertebral portion from each of the MIP image of the first region and the MIP image of the second region (S420).

The processor 13 may infer an inference value for each vertebral level from the MIP image of the first region and the MIP image of the second region, in which only the vertebral portion remains, using an AI-based detection model (S430).

The processor 13 may determine a final slice through the inference value (S440).

Here, the first region may be a sagittal plane region, and the second region may be a coronal plane region.

Here, the detection model may include a first detection model for the first region corresponding to the sagittal plane region and a second detection model for the second region corresponding to the coronal plane region.

The processor 13 may find a specific vertebral level from the MIP image of the first region and the MIP image of the second region, in which only the vertebral portion remains, and may determine the final slice using an inference value of the first detection model and an inference value of the second detection model for the specific vertebral level.

Referring to FIG. 5, the processor 13 may divide an MIP image 51 into a sagittal plane region and a coronal plane region to generate an MIP image 52 of the sagittal plane region and an MIP image 53 of the coronal plane region.

The processor 13 may input the MIP image 52 of the sagittal plane region to the segmentation model to remove artifacts that interfere with the detection model and may generate a first MIP image 54 of the sagittal plane region, from which only a vertebral bone region is extracted.

The processor 13 may input the MIP image 53 of the coronal plane region to the segmentation model to remove artifacts that interfere with the detection model and may generate a second MIP image 55 of the coronal plane region, from which only a vertebral bone region is extracted.

The processor 13 may input the first MIP image 54 and the second MIP image 55 to the first detection model and the second detection model, respectively, to return a candidate location of an expected slice for each of an image of the sagittal plane region and an image of the coronal plane region.

At this time, a weighted average may be calculated according to confidence scores of the first detection model and the second detection model to determine a final location of the slice, thereby generating a final slice image 36.

Although FIG. 4 illustrates that the respective steps are sequentially performed, this is merely for illustratively describing the technical spirit of the present embodiment. Accordingly, those skilled in the art may variously modify and apply the embodiment by changing the order described in FIG. 4 or by performing the respective steps in parallel without departing from the essential characteristics of the present embodiment. Therefore, FIG. 4 is not limited to a chronological order.

Meanwhile, in the above description, the steps illustrated in FIG. 4 may be further divided into additional steps or combined into fewer steps according to an implementation of the present disclosure. Further, some steps may be omitted as necessary, and the order of the respective steps may be changed.

Meanwhile, the disclosed embodiments may be implemented in the form of a recording medium storing instructions executable by a computer. The instructions may be stored in the form of program code, and when executed by a processor, may generate program modules to perform operations of the disclosed embodiments. The recording medium may be implemented as a computer-readable recording medium.

The computer-readable recording medium may include all types of recording media storing instructions readable by a computer. For example, the recording medium may include ROM (Read Only Memory), RAM (Random Access Memory), a magnetic tape, a magnetic disk, a flash memory, and an optical data storage device.

Although the disclosed embodiments have been described above with reference to the accompanying drawings, those skilled in the art to which the present disclosure pertains will understand that the present disclosure may be implemented in forms different from the disclosed embodiments without changing the technical spirit or essential characteristics of the present disclosure. Therefore, the disclosed embodiments are illustrative and should not be construed as limiting.

## Claims

1. An apparatus for measuring muscles by region for an L3 slice in a CT image, comprising:
a memory configured to store at least one process for performing measurement of muscles by region for the L3 slice in the CT image; and
a processor configured to perform an operation according to the process,
wherein the processor is configured to:
generate a first muscle mask and a second muscle mask, respectively;
indicate an intersection region of the first muscle mask and the second muscle mask as an actual muscle; and
divide the actual muscle into a plurality of regions and represent a muscle quality for each region.

2. The apparatus of claim 1,
wherein the first muscle mask is extracted from an L3 slice image based on a first model for segmenting an entire muscle,
wherein the second muscle mask is extracted from the L3 slice image based on a second model for segmenting muscles of detailed regions.

3. The apparatus of claim 1,
wherein the plurality of regions comprise:
an anterior muscle, a lateral muscle, a posterior muscle, and a psoas muscle.

4. The apparatus of claim 2,
wherein the first model and the second model have different ranges of regions of interest from each other.

5. A method for detecting a vertebral level in an AI-based MIP image, performed by a processor of an apparatus, the method comprising:
generating a first muscle mask and a second muscle mask, respectively;
indicating an intersection region of the first muscle mask and the second muscle mask as an actual muscle; and
dividing the actual muscle into a plurality of regions and representing a muscle quality for each region,
wherein the method is for measuring muscles by region for an L3 slice in a CT image.

6. An apparatus for AI-based vertebral level detection in an MIP image, comprising:
a memory configured to store at least one process for performing AI-based vertebral level detection in the MIP image; and
a processor configured to perform an operation according to the process,
wherein the processor is configured to:
divide the MIP image into a first region and a second region using an AI-based segmentation model;
remove portions other than a vertebral portion from each of the MIP image of the first region and the MIP image of the second region;
infer an inference value for each vertebral level from the MIP image of the first region and the MIP image of the second region, in which only the vertebral portion remains, using an AI-based detection model; and
determine a final slice through the inference value.

7. The apparatus of claim 6,
wherein the first region is a sagittal plane region,
wherein the second region is a coronal plane region.

8. The apparatus of claim 6,
wherein the detection model comprises:
a first detection model for the first region; and
a second detection model for the second region.

9. The apparatus of claim 8,
wherein the processor is configured to:
find a specific vertebral level from the MIP image of the first region and the MIP image of the second region, in which only the vertebral portion remains; and
determine the final slice using an inference value of the first detection model for the specific vertebral level and an inference value of the second detection model for the specific vertebral level.

10. A method for detecting a vertebral level in an AI-based MIP image, performed by a processor of an apparatus, the method comprising:
dividing the MIP image into a first region and a second region using an AI-based segmentation model;
removing portions other than a vertebral portion from each of the MIP image of the first region and the MIP image of the second region;
inferring an inference value for each vertebral level from the MIP image of the first region and the MIP image of the second region, in which only the vertebral portion remains, using an AI-based detection model; and
determining a final slice through the inference value.
